# EUROPEAN PATENT APPLICATION

(11) **EP 0 726 079 A2**
(43) Date of publication of application: **14.08.1996**
(21) Application number: 96300862.8
(22) Date of filing: 08.02.1996
(51) Int. Cl.: A61M 3/02

(54) **Nozzle for enema container**

(30) Priority: 08.02.1995 GB 9502417
(71) Applicant: C.B. FLEET COMPANY, INC., Lynchburg, Virginia 24506 (US)
(72) Inventor: O'Reagan, James Richard, Forest, Virginia 24551 (US)
(74) Representative: Hillier, Peter

(57) **Abstract**

A nozzle element has a hollow nozzle (10) having an orifice (14) at a forward end (12) thereof. At the base of the nozzle element a flange (18) extends away from a side wall (20) of the nozzle to form a collar of greater breadth than the nozzle. A container of deformable material is attached to the base of the nozzle (10). The interior of the container communicates with the hollow interior of the nozzle to deliver enema solution in use from the container through the nozzle and out from said orifice (14).

## Description

The invention relates to a nozzle particularly suitable for use with an enema container but not exclusively so, and a ready-to-use enema dispenser comprising nozzle and container.

It is known to provide "ready to use" enema dispensers comprising a deformable container having a nozzle, the container being of a material which can be easily deformed to discharge enema solution therein through the nozzle, the nozzle being of a pliant material. Constructions for nozzles for such ready-to-use enemas are disclosed in United States Patents Nos. 2869545 (Forsyth) and 3486503 (Porter et al). In both of these there is disclosed a nozzle formed in two pieces, the first being the flexible/pliant nozzle portion tapered in shape and the second being a screw cap which receives the nozzle and secures the nozzle to the enema container. In each of the devices disclosed there is a third component in the form of a releasable cap covering most of the nozzles tapered surface and which permits that portion of the nozzle covered to be ready provided with a lubricant for ease of insertion in immediate use and provides some protection against contamination of the nozzle before use. The nozzle, of gently tapering configuration, is flared out towards the part where it interconnects with the screw cap.

This flare does provided some protection against excessive insertion of the nozzle in use though this is far from ideal. Also, the flared shoulder does little to prevent backflow of the enema solution from the patent before it has had time to take effect.

In use, especially if lubricant is used, the enema container can become slippery and difficult to use.

The invention provides in one of its aspects a ready-to-use enema dispenser comprising:
a nozzle portion having a hollow elongate nozzle and, at a forward end, an orifice through which enema solution may in use be dispensed, the nozzle portion having at a rearward end an annular flange forming a collar of larger breadth than the nozzle; and
a container of deformable material attached to the base of said nozzle portion, the interior of the container communicating with the hollow interior of said nozzle to deliver enema solution in use from the interior of the container through the nozzle and out from said orifice.

The present invention provides in another of its aspects a nozzle element for an enema dispenser container comprising an elongate hollow nozzle having at a forward end a dispensing orifice and, at a rearward end, an annular flange forming a collar of larger breadth than the nozzle and at a rearmost end means for liquid-tight connection of the nozzle to a container whose contents are in use to be discharged through the nozzle.

The flange has three main functions. Firstly, it acts as a guard against excessive insertion. Secondly, it acts as a finger guard for the user. In some circumstances the containers themselves can become somewhat slippery and the flange permits ease of insertion of the nozzle by a user's fingers pushing against a rear face of the flange. Thirdly, the flange acts to some extent as a seal against back-flow while the contents of the container are being discharged through the nozzle.

The annular flange preferably slopes a little towards the rear of the nozzle portion.

The annular flange is preferably circular as viewed along an axial centre line of the nozzle, but it may be asymmetric.

The forward part of the nozzle may be provided with a cap to protect the nozzle against contamination and also to allow the nozzle to be coated with lubricant and/or medicated substances so that on removal of the cap the enema is ready for immediate use.

Preferably the means for attachment to the container is a female-threaded socket, desirably having finger grips on an outer circumferential surface thereof, for co-operation with a male-threaded end of a deformable container (in the manner of toothpaste tube).

Desirably the nozzle, flange and screw threaded socket are made in one piece. The material of the nozzle is preferably slightly pliant and having a rounded tip at the forward, orifice end. The socket is desirably of thicker wall thickness than the nozzle in order to give it sufficient stiffness such that the container can be easily threadedly attached to it, without bursting the threads. Desirably the flange is disposed between the socket and the orifice end of the nozzle such that the socket is just to the rear of the flange.

The whole assembly is preferably made of plastics material, desirably the container is made of polyethylene, and the nozzle of ethylene vinyl acetate.

An embodiment of the invention will now be described, by way of example only, with reference to the accompanying drawings in which:
Fig 1 is a schematic cross-sectional view partly broken away showing a nozzle according to the invention;
Fig 2 is a schematic perspective view of the nozzle from above and one side shown with a cover and with a complementary container shown detached therefrom.
Fig. 3 is a schematic perspective view of an enema dispenser formed in one piece; from one side and below.

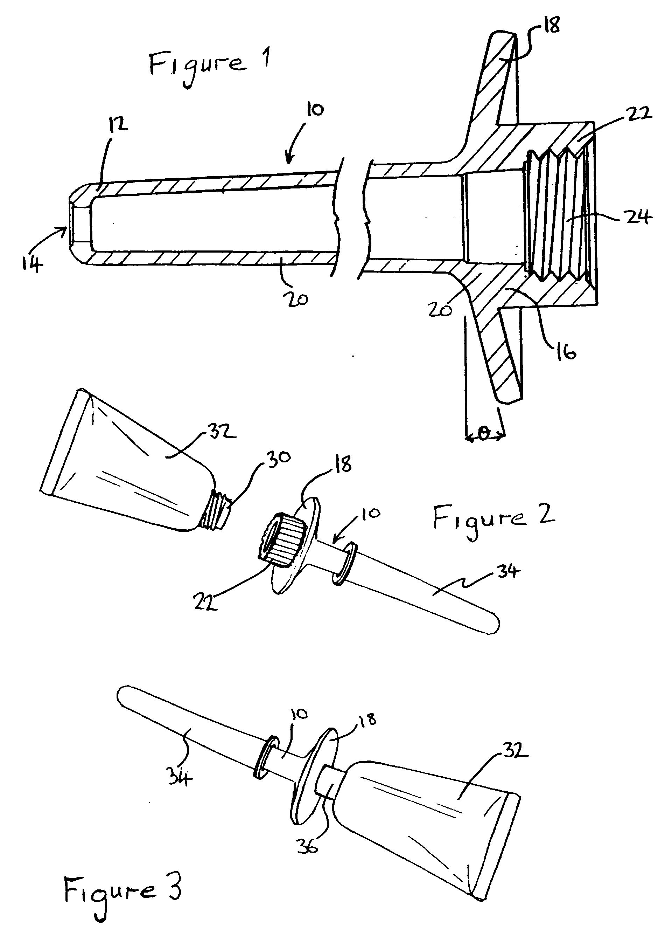

The nozzle element shown in Fig. 1 comprises an elongate slightly tapered, hollow nozzle 10 having, at a forward end 12, an orifice 14. At a rearward end 16 at the base of nozzle 10 the nozzle element has a flange 18, circular in shape as viewed along an axial centre line, and extending away from the wall 20 of the nozzle to form a collar of greater breadth than the nozzle. The flange also slopes rearwardly, being inclined at a small angle Q from the perpendicular to an axial centre line of the nozzle. Also at the rearward end of the nozzle element, just to the rear of the flange, there is provided an internally threaded (female) socket 22 having threads 24 cooperable with an externally threaded boss 30 (Fig. 2) provided on the neck of a container 32 (Fig. 2) in the manner of a toothpaste tube for liquid tight engagement therewith. Thus when container 32 is engaged with the nozzle element 1, liquid in the container 32 can be dispensed through orifice 14 by squeezing container 32. Fig. 2 shows the nozzle element 10 provided with a closure cap 34 used to seal the nozzle before use. A lubricant (not shown) is provided on the nozzle 10 but under closure cap 34 ready for immediate use of the dispenser.

In use the nozzle element 10 and container 32 are ready assembled with enema solution within the container. The closure cap 34 is removed and the nozzle 10 inserted into the anus of the patient. Flange 18 acts as a finger guard for the user, assists non-slip insertion prior to squeezing the container 32 for dispensing of the enema solution and helps to limit premature back-flow of solution.

Fig. 3 shows a small one-piece moulded enema dispenser incorporating all the elements of the invention, the dispenser in this embodiment not being provided with a screw threaded connection between the container and the nozzle element but joined by a tube 36. Corresponding labels have been used in Fig. 3 to indicate similar elements to those of the embodiment of Figs. 1 and 2. Small dispensers of this kind are often referred to as micro-enemas.

The nozzle and enema dispenser shown in the figures are made from ethylene vinyl acetate and from polyethylene plastics material respectively, and of such thickness and toughness that the nozzle 10 is slightly pliant and such that the container is tough enough to be squeezed to expel its contents without rupturing. Any other plastics or suitable other material may be used.

The nozzle could be rigid. The threads 24 may be arranged to be compatible with threads provided on a syringe body.

The flange need not be circular. the flange need not be symmetrically disposed relative to the nozzle.

## Claims

1. A ready-to-use enema dispenser comprising:
a nozzle portion having a hollow elongate nozzle (10) and, at a forward end (12), an orifice (14) through which enema solution may in use be dispensed; and
a container (32) of deformable material attached to the base of said nozzle portion, the interior of the container communicating with the hollow interior of said nozzle to deliver enema solution in use from the interior of the container through the nozzle and out from said orifice, and characterised by the nozzle portion having at a rearward end an annular flange (18) forming a collar of larger breadth than the nozzle.

2. A nozzle element for an enema dispenser container comprising an elongate hollow nozzle (10) having at a forward end (12) a dispensing orifice (14) and, at a rearmost end, means (22) for liquid-tight connection of the nozzle to a container (32) whose contents are in use to be discharged through the nozzle, characterised by the nozzle having, at a rearward end, an annular flange (18) forming a collar of larger breadth than the nozzle.

3. A nozzle element as claimed in claim 2, wherein the means (22) for attachment to the container (32) is a female-threaded (24) socket having finger grips on an outer circumferential surface thereof, for co-operation with a male-threaded end (30) of a deformable container.

4. A nozzle element as claimed in claim 3, wherein the nozzle (10), flange (18) and screw threaded socket (22) are made in one piece.

5. A nozzle element as claimed in claim 3 or 4, wherein the socket (22) is of thicker wall thickness than the nozzle (10).

6. A nozzle element as claimed in any one of claims 2 to 5, wherein the flange (18) is disposed between the socket (22) and the orifice end (12) of the nozzle such that the socket is just to the rear of the flange.

7. An enema dispenser or nozzle element as claimed in any preceding claim, wherein the annular flange (18) slopes towards the rear of the nozzle portion.

8. An enema dispenser or nozzle element as claimed in any preceding claim, wherein the annular flange (18) is circular as viewed along an axial centre line of the nozzle (10).

9. An enema dispenser or nozzle element as claimed in any preceding claim, wherein the forward part of the nozzle (10) is provided with a cap (34).

10. An enema dispenser or nozzle element as claimed in any preceding claim, wherein the material of the nozzle (10) is slightly pliant and has a rounded tip at the forward, orifice end (12).
